# EUROPÄISCHE PATENTSCHRIFT

(11) **EP 3 515 278 B1**
(45) Veröffentlichungstag und Bekanntmachung des Hinweises auf die Patenterteilung: **16.09.2020**
(21) Anmeldenummer: 17800788.6
(22) Anmeldetag: 08.11.2017
(51) Int. Cl.: A61B 1/00, A61B 1/07

(54) **ENDOSKOP**
ENDOSCOPE
ENDOSCOPE

(30) Priorität: 16.12.2016 DE 102016124731
(43) Veröffentlichungstag der Anmeldung: 31.07.2019
(73) Patentinhaber: OLYMPUS Winter & Ibe GmbH, 22045 Hamburg (DE)
(72) Erfinder: JÜRGENS, Thorsten, 20359 Hamburg (DE)
(74) Vertreter: Noack, Andreas
(86) Internationale Anmeldenummer: PCT/EP2017/078627
(87) Internationale Veröffentlichungsnummer: WO 2018/108396

(56) Entgegenhaltungen:
- JP-B2- 3 290 700

## Beschreibung

Die Erfindung betrifft ein Endoskop mit einem Hauptkörper, einem Schaft und einem den Schaft vom Hauptkörper bis zu einem distalen Ende des Schafts durchlaufenden Lichtleitfaserbündel, wobei das Lichtleitfaserbündel am distalen Ende des Schafts an einem Beleuchtungsaustritt endet, und das Lichtleitfaserbündel am Hauptkörper an einem Lichtleitstutzen zum Anschluss eines Lichtleitkabels endet.

Entsprechende Endoskope werden seit langem erfolgreich eingesetzt, um schwer zugängliche Hohlräume in technischen Anlagen oder in tierischen oder menschlichen Patienten optisch zu untersuchen. Dabei dient das Endoskop dazu, ein Bild von der inneren Oberfläche des Hohlraums aufzunehmen und außerhalb des Hohlraums zur Verfügung zu stellen. Die Beleuchtung über ein im Inneren des Endoskops verlaufendes Lichtleitfaserbündel. In vielen Fällen ist die zur Beleuchtung verwendete Lichtquelle als separates Gerät ausgeführt, das Licht wird dann über in ein Lichtleitkabel zu dem Endoskop geleitet.

Um eine möglichst hohe Flexibilität im Einsatz von Endoskopen zu erreichen, ist das Lichtleitkabel oftmals zur lösbaren Verbindung mit dem Endoskop ausgeführt. Dazu weist das Endoskop einen Lichtleitstutzen auf, auf welchen das Lichtleitkabel aufgesteckt und fixiert wird. Die Fixierung erfolgt zumeist über standardisierte Einrichtungen wie Bajonettkupplungen, Schnappkupplungen oder Gewinde.

Das Lichtleitfaserbündel kann direkt bis zu einer Stirnfläche des Lichtleitstutzens geführt sein. Oftmals kommt aber zur Anpassung der numerischen Apertur zwischen den Lichtleitfasern des Lichtleitkabels und den Lichtleitfasern des Lichtleitfaserbündels ein Faserkegel zum Einsatz.

Am distalen Ende des Schafts kann das Lichtleitfaserbündel direkt bis zu einem Beleuchtungsaustritt geführt sein. In manchen Endoskopen sind zwischen dem Lichtleitfaserbündel und dem Beleuchtungsaustritt zusätzliche optische Elemente zur Anpassung der Lichtaustrittsrichtung oder der Intensitätsverteilung vorgesehen, wie z.B. Linsen, Spiegel, Prismen oder verschmolzene Faserelemente.

Für unterschiedliche Anwendungen sind eine Vielzahl spezieller Endoskope entwickelt worden, welche sich beispielsweise hinsichtlich Schaftdurchmesser, Schaftlänge und Blickrichtung unterscheiden. Je nach Ausführung und Anwendungsfall weisen dabei die in den Endoskopen verlegten Lichtleitfaserbündel unterschiedliche Anzahlen von Lichtleitfasern und somit auch unterschiedliche Durchmesser auf.

Um nicht gleichzeitig eine große Anzahl unterschiedlicher Lichtleitkabel vorhalten zu müssen, werden Endoskope gemeinsam mit wenigen standardisierten Lichtleitkabeln verwendet. Um dabei stets eine vollständige Ausleuchtung des im Endoskop verlegten Lichtleitfaserbündels zu gewährleisten, weisen die Lichtleitkabel stets einen lichtleitenden Querschnitt auf, der größer oder gleich der am Lichtleitstutzen verfügbaren Querschnittsfläche des Lichtleitfaserbündels ist.

Wird nun ein Endoskop mit einem Lichtleitkabel verwendet, dessen lichtleitender Querschnitt größer ist als die am Lichtleitstutzen verfügbare Querschnittsfläche des Lichtleitfaserbündels, so wird ein Teil des Beleuchtungslichts nicht in das Lichtleitfaserbündel eingekoppelt, sondern trifft auf das Material des Lichtleitstutzens und wird hier zu einem großen Teil absorbiert. Dies führt zu einer Erwärmung des Hauptkörpers des Endoskops, welche unerwünscht ist.

Das Problem wird bei modernen Endoskopiesystemen dadurch verstärkt, dass durch die Verwendung immer höherer Bildauflösungen die benötigte Beleuchtungsintensität steigt. Dadurch steigt auch die bei Querschnittsabweichungen absorbierte Energiemenge.

Es besteht daher die Aufgabe der Erfindung darin, ein Endoskop bereitzustellen, welches hinsichtlich der beschriebenen Problematik verbessert ist.

Ein Endoskop gemäß dem Oberbegriff des Anspruchs 1 lehrt die JP 3290700.

Diese Aufgabe wird erfindungsgemäß gelöst durch ein Endoskop gemäß Anspruch 1. Durch die verminderte Absorption im Material des Lichtleitstutzens wird die unerwünschte Erwärmung des Hauptkörpers reduziert.

In einer möglichen Ausgestaltung eines erfindungsgemäßen Endoskops umfasst die Stirnfläche des Lichtleitstutzens eine Koppelfläche, durch welche Licht in das Lichtleitfaserbündel eingekoppelt werden kann, und ferner eine Blindfläche, durch welche Licht nicht in das Lichtleitfaserbündel eingekoppelt werden kann, und die Blindfläche ist reflektierend ausgeführt. Durch die reflektierend ausgeführte Blindfläche wird licht, welches vom Lichtleitkabel im Bereich der Blindfläche abgegeben wird, reflektiert und in das Lichtleitkabel zurückgeleitet. Das Licht gelangt so zurück zur Lichtquelle, wo es zwar ebenfalls absorbiert wird. Allerdings ist die durch diese Absorption im Bereich der Lichtquelle auftretende Erwärmung deutlich weniger störend als eine Erwärmung des Hauptkörpers des Endoskops.

In einer bevorzugten Weiterbildung eines erfindungsgemäßen Endoskops weist die Blindfläche in einem Wellenlängenbereich von 350nm bis 950nm einen Reflexionsgrad von größer 85%, insbesondere größer 90%, vorzugsweise insbesondere größer 95%, auf. Dies ist im Sinne der Erfindung so zu verstehen, dass die Blindfläche bei jeder Wellenlänge in dem genannten Wellenlängenbereich wenigstens den angegebenen Reflexionsgrad aufweist.

Gemäß einer Ausführung eines erfindungsgemäßen Endoskops umfasst der Lichtleitstutzen eine das Lichtleitfaserbündel einhüllende Hülse, und dass die Stirnfläche der Hülse ist mit einer reflektierenden Beschichtung versehen.

Die reflektierende Beschichtung kann ein Lack ist oder einen Lack umfassen. Beispielsweise kann die Stirnfläche der Hülse mit einer dünnen Schicht eines hochreflektierenden Metalls bedampft sein, welche wiederum durch eine dünne Schicht eines schützenden Lacks bedeckt ist.

In der Ausführung eines erfindungsgemäßen Endoskops umfasst die Stirnfläche des Lichtleitstutzens ein Deckglas, welches im Bereich der Blindfläche eine reflektierende Beschichtung aufweist. Dabei kann die reflektierende Beschichtung auf der in Richtung des Lichtleiterstutzens weisenden Seite des Deckglases aufgebracht sein, so dass sie vor mechanischer Beschädigung beim Montieren oder Demontieren des Lichtleitkabels geschützt ist.

Das Deckglas kann ferner im Bereich der Koppelfläche eine reflexionsmindernde Beschichtung aufweisen. Dadurch wird gleichzeitig die Effizienz der Lichteinkopplung optimiert.

Bei der reflektierenden und/oder der reflexionsmindernden Beschichtung des Deckglases kann es sich beispielsweise um eine mehrlagige dielektrische Beschichtung handeln. Mittels entsprechender Beschichtungen lassen sich über große Wellenlängenbereiche hohe Transmissions- oder Reflexionsgrade erreichen.

Die Erfindung wird im Folgenden anhand einiger beispielhafter Figuren erläutert. Es zeigen:
Fig. 1: ein Endoskopiesystem,
Fig. 2: ein Lichtleitstutzen mit aufgesetztem Lichtleitkabel,
Fig. 3: ein weiterer Lichtleiterstutzen mit aufgesetztem Lichtleitkabel

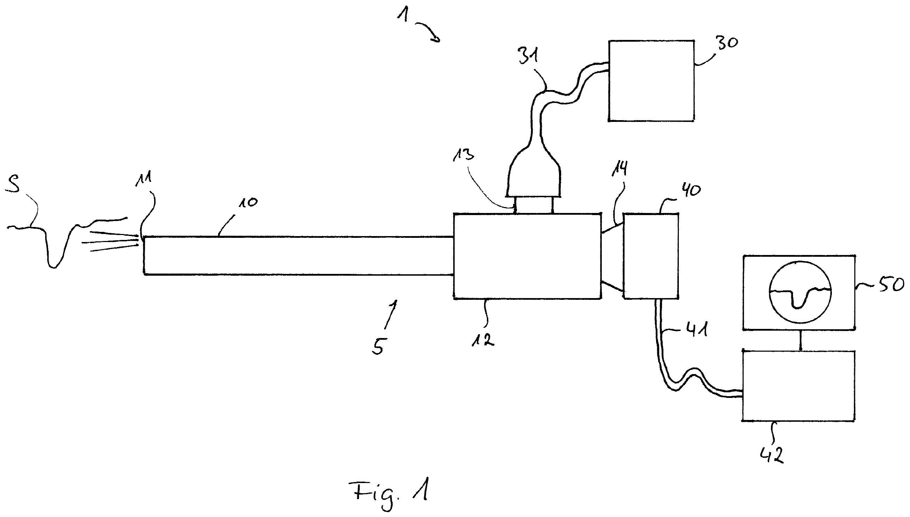

Figur 1 zeigt ein Endoskopiesystem 1 mit einem Endoskop 5. Das Endoskop 5 umfasst einen Schaft 10 mit einem Beleuchtungsaustritt 11, einen Hauptkörper 12, einen Lichtleiterstutzen 13 sowie einen Okulartrichter 14.

Eine Lichtquelle 30 ist über ein Lichtleitkabel 31 mit dem Endoskop 5 verbunden. Dazu ist das Lichtleitkabel 31 auf den Lichtleitstutzen 13 aufgesteckt. Licht von der Lichtquelle 30 wird über das Lichtleitkabel 31 in ein nicht dargestelltes Lichtleitfaserbündel im Endoskop 5 eingekoppelt und in diesem Lichtleitfaserbündel zu dem Beleuchtungsaustritt 11 am distalen Ende des Schaftes 10 geleitet. Dort wird das Licht in Richtung einer anatomischen Struktur S abgestrahlt.

Das von der anatomischen Struktur S zurückgeworfene Licht wird über ein nicht dargestelltes Objektiv am distalen Ende des Schaftes 10 gesammelt und über einen ebenfalls nicht dargestellten Bildleiter, bei dem es sich um ein Relais-Linsensystem oder ein Bildfaserbündel handeln kann, zum Okulartrichter 14 geleitet. Dort wird es von einer auf dem Okulartrichter 14 montierten Kamera 40 in ein Videobild umgewandelt und über ein Videokabel 41 an eine Bildverarbeitungseinrichtung 42 geleitet, welche das Videobild zur Darstellung auf einen Monitor 50 leitet.

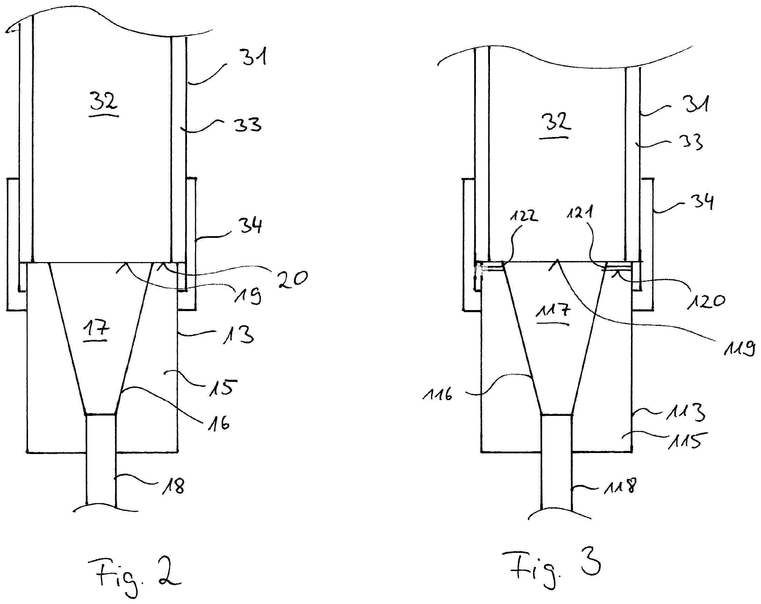

In Figur 2 ist der Lichtleitstutzen 13 des Endoskops 5 in einer prinzipiellen Schnittdarstellung gezeigt. Er besteht aus einer Hülse 15 mit einer sich konisch aufweitenden Bohrung 16, in welche ein Faserkegel 17 eingefasst ist. An den Faserkegel 17 angrenzend ist ein Lichtleitfaserbündel 18 in die Bohrung 16 eingesetzt.

Auf den Lichtleitstutzen 13 ist das Lichtleitkabel 31 aufgesetzt. Das Lichtleitkabel umfasst einen lichtleitenden Querschnitt 32 aus einzelnen Lichtleitfasern, die der Übersichtlichkeit halber nicht dargestellt sind. Das Lichtleitkabel ist von einem Schutzschlauch 33 umgeben, welcher die Fasern vor mechanischen Beschädigungen schützt. Auf dem Schutzschlauch 33 ist eine Steckhülse 34 befestigt, mit welcher das Lichtleitkabel 31 auf den Lichtleitstutzen 13 aufgesteckt und fixiert wird.

Licht aus dem Lichtleitkabel 31 wird in den Faserkegel 17 eingekoppelt und von diesem an das Lichtleitfaserbündel 18 weitergeleitet. Dabei sorgt die konische Form des Faserkegels für eine Anpassung der numerischen Apertur der Fasern des Lichtleitkabels 31 an die numerische Apertur der Fasern des Faserbündels 18.

Die in Richtung des Lichtleitkabels 31 weisende Endfläche des Faserkegels 17 stellt eine Koppelfläche 19 dar, durch welche Licht in den Faserkegel 17 und somit in das Lichtleitfaserbündel 18 eingekoppelt werden kann. Die in Richtung des Lichtleitkabels 31 weisende Stirnfläche der Hülse 15 stellt hingegen eine ringförmige Blindfläche 20 dar, durch welche eine Lichteinkopplung nicht möglich ist.

Licht, welches von dem Lichtleitkabel 31 im Bereich der Blindfläche 20 abgegeben wird, wir zu einem großen Teil von der Hülse 15 absorbiert, so dass sich diese erwärmt. Im dargestellten Beispiel ist der Durchmesser des lichtleitenden Querschnitts 32 des Lichtleitkabels 31 etwa 50% größer als der Durchmesser der Koppelfläche 19. Bei dieser Konstellation gehen bereits etwa 50% des durch das Lichtleitkabel 31 transportierten Lichts verloren. Diese treffen auf die Blindfläche 20 und werden dort zu einem großen Teil absorbiert und tragen nur zur Erwärmung der Hülse 15 und somit des Hauptkörpers 12 bei.

In Figur 3 ist ein modifizierter Lichtleitstutzen 113 eines Endoskops gemäß der Erfindung dargestellt. Der Lichtleitstutzen 113 entspricht im Wesentlichen dem Lichtleitstutzen 13 der Figur 2. Sich entsprechende Komponenten sind daher mit einem um 100 erhöhten Bezugszeichen versehen und nicht erneut beschrieben. Auf dem Lichtleitstutzen 113 ist wiederum das Lichtleitkabel 31 aufgesetzt und wird hier ebenfalls nicht weiter beschrieben.

Auf der Blindfläche 120 der Hülse 115 sind hier eine hochreflektierende Metallschicht 121 und eine schützende Lackschicht 122 aufgebracht. Licht, welches im Bereich der Blindfläche 120 aus dem Lichtleitkabel 31 abgegeben wird, trifft nun auf die reflektierende Schicht 121 und wird in Richtung des Lichtleitkabels 31 zurück reflektiert. Bis auf geringe Koppelverluste wird dieses Licht durch das Lichtleitkabel 31 zurück zur Lichtquelle 30 transportiert und dort abgegeben. Zwar wird auch hier das reflektierte Licht letztlich in Wärme umgewandelt, allerdings ist dies im Bereich der Lichtquelle 30 weite weniger kritisch als im Bereich des Lichtleiterstutzens 113.

Die Metallschicht 121 kann beispielsweise eine Silberschicht sein. Mit einer solchen Schicht können über einen weiten Wellenlängenbereich Reflexionsgrade von über 85% erreicht werden.

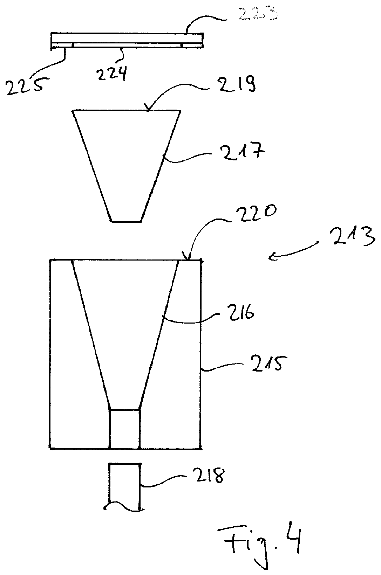

In Figur 4 ist ein weiterer Lichtleitstutzen 213 eines Endoskops gemäß der Erfindung in einer Explosionsdarstellung gezeigt. Der Lichtleitstutzen 213 umfasst wiederum eine Hülse 215 mit konischer Bohrung 216. In die Bohrung ist von einer Seite ein Faserkegel 217 eingesetzt, von der anderen Seite das Ende eines in dem Endoskop verlegten Lichtleitfaserbündels 218. Auf der Stirnseite der Hülse 215 und des Faserkegels 217 ist ein Deckglas 223 angeordnet, auf dessen der Hülse 215 zugewandte Seite im Bereich der Koppelfläche 219 eine reflexionsmindernde Schicht 224 aufgebracht ist. Im Bereich der Blindfläche 220 ist auf dem Deckglas 223 eine reflektierende Schicht 225 aufgebracht.

Die Wirkungsweise des Lichtleitstutzens 213 entspricht im Wesentlichen der des Lichtleitstutzens113 aus Figur 3, auf eine erneute Darstellung wird daher verzichtet.

Die Beschichtungen 224, 225 können als mehrlagige dielektrische Beschichtungen ausgeführt sein, um über einen möglichst weiten Wellenlängenbereich eine ausreichende Wirksamkeit zu gewährleisten. Für die reflektierende Schicht 225 kann mit einer dielektrischen Beschichtung über einen weiten Wellenlängenbereich ein Reflexionsgrad von über 95% erreicht werden.

Die reflektierenden Schichten aller Ausführungsbeispiele sind so ausgelegt, dass der gewünschte Reflexionsgrad möglichst im gesamten von der Lichtquelle abgegebenen Spektrum erreicht wird. Dies umfasst den gesamten Bereich des sichtbaren Lichts, aber auch den nahen UV- und IR-Bereich. Diese Wellenlängenbereiche werden insbesondere für Fluoreszenzuntersuchungen benötigt und daher von der Lichtquelle mit erzeugt. Entsprechend soll auch die Absorption der entsprechenden spektralen Anteile des Lichts im Lichtleiterstutzen vermieden werden.

## Patentansprüche

1. Endoskop mit einem Hauptkörper (12), einem Schaft (10) und einem den Schaft (10) vom Hauptkörper (12) bis zu einem distalen Ende des Schafts durchlaufenden Lichtleitfaserbündel (18), wobei das Lichtleitfaserbündel (18,118,218) am distalen Ende des Schafts (10) an einem Beleuchtungsaustritt (11) endet, und das Lichtleitfaserbündel (18) am Hauptkörper (12) an einem Lichtleitstutzen (13,113,213) zum Anschluss eines Lichtleitkabels (31) endet, wobei der Lichtleitstutzen (13,113,213) ausgeführt ist, um eine Absorption von Licht, welches von dem Lichtleitkabel (31) nicht in das Lichtleitfaserbündel (18,118,218) eingekoppelt werden kann, durch das Material des Lichtleitstutzens (13) zu reduzieren,
wobei die Stirnfläche des Lichtleitstutzens (113,213) eine Koppelfläche (119,219) umfasst, durch welche Licht in das Lichtleitfaserbündel (118,218) einkoppelbar ist, und wobei die Stirnfläche des Lichtleitstutzens (13) ferner eine Blindfläche (120,220) umfasst, durch welche Licht nicht in das Lichtleitfaserbündel (18) einkoppelbar ist, und wobei die Blindfläche (120,220) reflektierend ausgeführt ist,
**dadurch gekennzeichnet, dass** die Stirnfläche des Lichtleitstutzens (213) ein Deckglas (223) umfasst, welches im Bereich der Blindfläche (220) eine reflektierende Beschichtung (225) aufweist.

2. Endoskop nach Anspruch 1, **dadurch gekennzeichnet, dass** die Blindfläche (120,220) in einem Wellenlängenbereich von 350nm bis 950nm einen Reflexionsgrad von größer 85%, insbesondere größer 90%, vorzugsweise insbesondere größer 95%, aufweist.

3. Endoskop nach Anspruch 1 oder 2, dadurch gelkennzeichnet, dass der Lichtleitstutzen (113) eine das Lichtleitfaserbündel (118) einhüllende Hülse (115) umfasst, und dass die Stirnfläche der Hülse (115) mit einer reflektierenden Beschichtung (121,122) versehen ist.

4. Endoskop nach Anspruch 3, **dadurch gekennzeichnet, dass** die reflektierende Beschichtung (121,122) ein Lack ist oder einen Lack umfasst.

5. Endoskop nach einem der Ansprüche 1 bis 4, **dadurch gekennzeichnet, dass** das Deckglas (223) im Bereich der Koppelfläche (219) eine reflexionsmindernde Beschichtung (224) aufweist.

6. Endoskop nach einem der Ansprüche 1 bis 5, **dadurch gekennzeichnet, dass** das Deckglas (233) eine mehrlagige dielektrische Beschichtung (224,225) aufweist.

## Claims

1. Endoscope comprising a main body (12), a shaft (10) and an optical fiber bundle (18) which extends through the shaft (10) from the main body (12) to a distal end of the shaft, the optical fiber bundle (18, 118, 218) terminating, at the distal end of the shaft (10), at an illumination outlet (11), and the optical fiber bundle (18) terminating, at the main body (12), at an optical connector (13, 113, 213) for connecting an optical cable (31), the optical connector (13, 113, 213) being designed to reduce the amount of light that cannot be coupled into the optical fiber bundle (18, 118, 218) from the optical cable (31) absorbed by the material of the optical connector (13),
the end face of the optical connector (113, 213) comprising a coupling surface (119, 219) by means of which light can be coupled into the optical fiber bundle (118, 218), and the end face of the optical connector (13) further comprising a blind surface (120, 220) by means of which light cannot be coupled into the optical fiber bundle (18), and the blind surface (120, 220) being reflective,
**characterized in that** the end face of the optical connector (213) comprises a cover glass (223) which has a reflective coating (225) in the region of the blind surface (220).

2. Endoscope according to claim 1, **characterized in that** the blind surface (120, 220) has a reflectance of greater than 85%, in particular greater than 90%, particularly preferably greater than 95%, in a wavelength range of from 350 nm to 950 nm.

3. Endoscope according to either claim 1 or claim 2, **characterized in that** the optical connector (113) comprises a sleeve (115) which surrounds the optical fiber bundle (118), and **in that** the end face of the sleeve (115) is provided with a reflective coating (121, 122).

4. Endoscope according to claim 3, **characterized in that** the reflective coating (121, 122) is a lacquer or comprises a lacquer.

5. Endoscope according to any of claims 1 to 4, **characterized in that** the cover glass (223) has a reflection-reducing coating (224) in the region of the coupling surface (219).

6. Endoscope according to any of claims 1 to 5, **characterized in that** the cover glass (233) has a multilayered dielectric coating (224, 225).

## Revendications

1. Endoscope comportant un corps principal (12), une tige (10) et un faisceau de fibres optiques (18) s'étendant à travers la tige (10) à partir du corps principal (12) jusqu'à une extrémité distale de la tige, le faisceau de fibres optiques (18, 118, 218) se terminant à l'extrémité distale de la tige (10) au niveau d'une sortie d'éclairage (11), et le faisceau de fibres optiques (18) se terminant sur le corps principal (12) au niveau d'un embout optique (13, 113, 213) pour raccorder un câble optique (31), l'embout optique (13, 113, 213) étant conçu pour réduire, au moyen du matériau de l'embout optique (13), l'absorption de lumière ne pouvant pas être injectée dans le faisceau de fibres optiques (18, 118, 218) à partir du câble optique (31),
la face frontale de l'embout optique (113, 213) comprenant une surface d'injection (119, 219) à travers laquelle la lumière peut être injectée dans le faisceau de fibres optiques (118, 218), et la face frontale de l'embout optique (13) comprenant en outre une surface aveugle (120, 220) à travers laquelle la lumière ne peut pas être injectée dans le faisceau de fibres optiques (18), et la surface aveugle (120, 220) étant conçue pour être réfléchissante,
**caractérisé en ce que** la face frontale de l'embout optique (213) comprend une lamelle couvre-objet (223), laquelle présente un revêtement réfléchissant (225) dans la zone de la surface aveugle (220).

2. Endoscope selon la revendication 1, **caractérisé en ce que** la surface aveugle (120, 220) présente une réflectance supérieure à 85 %, en particulier supérieure à 90 %, de préférence en particulier supérieure à 95 %, dans une plage de longueurs d'onde de 350 nm à 950 nm.

3. Endoscope selon la revendication 1 ou 2, **caractérisé en ce que** l'embout optique (113) comprend un manchon (115) enveloppant le faisceau de fibres optiques (118), et **en ce que** la face frontale du manchon (115) est pourvue d'un revêtement réfléchissant (121, 122).

4. Endoscope selon la revendication 3, **caractérisé en ce que** le revêtement réfléchissant (121, 122) est une laque ou comprend une laque.

5. Endoscope selon l'une des revendications 1 à 4, **caractérisé en ce que** la lamelle couvre-objet (223) présente un revêtement de réduction de réflexion (224) dans la zone de la surface d'injection (219).

6. Endoscope selon l'une des revendications 1 à 5, **caractérisé en ce que** la lamelle couvre-objet (233) présente un revêtement diélectrique multicouche (224, 225).
